# EUROPEAN PATENT APPLICATION

(11) **EP 3 744 829 A1**
(43) Date of publication of application: **02.12.2020**
(21) Application number: 19740768.7
(22) Date of filing: 17.01.2019
(51) Int. Cl.: C12N 1/20, C12Q 1/02

(54) **MEDIUM FOR CULTURING PNEUMOCOCCAL SAMPLES**

(30) Priority: 22.01.2018 JP 2018007925
(71) Applicant: THE RESEARCH FOUNDATION FOR MICROBIAL DISEASES OF OSAKA UNIVERSITY, Suita-shi, Osaka 565-0871 (JP)
(72) Inventor: PIAO, Zhenyu, Kanonji-shi, Kagawa 768-0065 (JP); KOIZUMI, Yuka, Kanonji-shi, Kagawa 768-0065 (JP); MIYATAKE, Hiroshi, Kanonji-shi, Kagawa 768-0065 (JP)
(74) Representative: Vossius & Partner Patentanwälte Rechtsanwälte mbB
(86) International application number: PCT/JP2019/001197
(87) International publication number: WO 2019/142848

(57) **Abstract**

An object of the present invention is to provide a technique capable of further increasing the absolute detection value and/or the ratio of the detection value to a reference in a pneumococcal antibody sample evaluation test, or a technique capable of evaluating pneumococcal antibody samples against a wider variety of pneumococcal strains.

The object is achieved by a medium for preparing a pneumococcal sample for use in a pneumococcal antibody sample evaluation test, the medium containing a solid medium.

## Description

### Technical Field

The present invention relates to a medium for culturing pneumococcal samples etc.

### Background Art

Current pneumococcal vaccines are polysaccharide-based vaccines in which a polysaccharide (capsule polysaccharide), which is a synthetic component of a capsule present on the bacterial surface, is an antigen. Examples of polysaccharide-based vaccines include pediatric 7-, 10-, and 13-valent pneumococcal conjugate vaccines (PCV7, PCV10, PCV13); and 23-valent polysaccharide vaccines (PPSV23). In pneumococcal infection, serotype-specific phagocyte bactericidal capacity of antibodies against capsular polysaccharides is the main infection protection mechanism. Thus, current pneumococcal vaccines have been put to practical use as vaccines that contain human pathogenic serotype pneumococcal capsular antigens. The introduction of routine immunization for children has significantly reduced invasive pneumococcal disease (IPD) caused by serotype strains contained in pediatric vaccines. However, with the spread of vaccines, serotype replacement has occurred, and the number of serotypes that cannot be covered by vaccines has increased. For this reason, there is a demand for the development of a novel pneumococcal vaccine that contains, as an antigen, a protein common in all pneumococcal strains.

Pneumococcal surface protein A (PspA) is known to serve as a virulence factor to inhibit the deposition of complement C3 onto the bacterial surface, thus evading the body's immunity. PspA is expressed in all pneumococcal strains, and shows cross-reactivity against different families. Thus, PspA is regarded as the most promising common antigen candidate for novel pneumococcal vaccines. The α-helical region and the proline-rich region of PspA are known to have antigen epitopes for recognition by antibodies having protective actions against pneumococcal infection. According to amino acid sequences of clade definition regions present at its N-terminal, PspA is roughly grouped into three families, including six subgroups, called clades. Regarding PspA family distribution, families 1 and 2 account for 98% or more of pneumococcal clinical isolates. It is considered that an anti-PspA-specific antibody is known to exert an antagonistic effect on the complement inhibitory activity of PspA, and activate complement C3 deposition, which induces opsonic activity, thus exhibiting a protective activity against infection of the bacteria. Development of an efficacy evaluation index that is highly relevant to the protective activity against infection is considered to be very important for the development of vaccines based on PspA.

It has been reported that current vaccines based on capsular antigens do not have a correlation between an ELISA (enzyme linked immunosorbent assay) antibody titer and protection ability against infection. Measurement of the amount of antibody having a function (opsonic activity) of promoting leukocyte phagocytosis is currently a surrogate marker for evaluating the efficacy of the vaccines.

Based on the above circumstances, it is desirable to develop a test system that can measure the functional antibodies of PspA vaccines for evaluating the efficacy of PspA vaccines. Known vaccine measurement methods have not been able to be used for evaluation of vaccines that use a PspA-containing protein as an antigen, and there is literature that has attempted to establish a method of evaluating the vaccines in some strains (Non-patent Literature 1 and 2). However, there is no report on evaluation using all clinical isolates.

### Citation List

### Non-patent Literature

NPL 1: Vaccine 29: 1634-1642, 2011.
NPL 2: Clin Vaccine Immunol 20: 1549-1558, 2013.

### Summary of Invention

### Technical Problem

An object of the present invention is to provide a technique capable of further increasing the absolute detection value and/or the ratio of the detection value to a reference in a pneumococcal antibody sample evaluation test, and/or a technique capable of evaluating pneumococcal antibody samples against a wider variety of pneumococcal strains. Preferably, an object of the present invention is to provide a technique capable of further increasing sensitivity of an evaluation test of vaccines in which a pneumococcal surface/intracytoplasmic protein such as PspA is an antigen, and/or a technique capable of evaluating pneumococcal antibody samples against a wider variety of pneumococcal strains.

### Solution to Problem

As a result of extensive research, the present inventors found that the above object can be attained by the use of, as a pneumococcal sample for use in a pneumococcal antibody sample evaluation test, a pneumococcal sample obtained by culturing a pneumococcus in a solid medium. Based on this finding, the inventors conducted further research, and accomplished the present invention. Specifically, the present invention includes the following aspects.
Item 1. A medium for preparing a pneumococcal sample for use in a pneumococcal antibody sample evaluation test, the medium containing a solid medium.
Item 2. The medium according to Item 1, wherein the solid medium contains agar.
Item 3. The medium according to Item 1 or 2, wherein the solid medium contains a blood component and/or catalase. Item 4. The medium according to any one of Items 1 to 3, wherein the pneumococcal antibody sample is collected from a subject to which a pneumococcal vaccine has been administered. Item 5. The medium according to Item 4, wherein the sample is a blood sample.
Item 6. The medium according to Item 4 or 5, wherein the pneumococcal vaccine contains at least one member selected from the group consisting of pneumococcal surface/intracytoplasmic proteins and pneumococcal capsular components as an antigen. Item 7. The medium according to any one of Items 4 to 6, wherein the pneumococcal vaccine contains a pneumococcal surface/ intracytoplasmic protein as an antigen.
Item 8. The medium according to any one of Items 1 to 7, wherein the evaluation test evaluates at least one property selected from the group consisting of a binding property of the pneumococcal antibody sample to the pneumococcal sample, a complement deposition ability based on the binding, and a bactericidal property based on the complement deposition. Item 9. The medium according to Item 8, wherein the evaluation test evaluates at least one property selected from the group consisting of the complement deposition ability and the bactericidal property.
Item 10. The medium according to Item 8 or 9, wherein the evaluation test evaluates the bactericidal property. Item 11. A composition for preparing the medium according to any one of Items 1 to 10, the composition containing a component for solidifying the medium.
Item 12. A kit for preparing the medium according to any one of Items 1 to 10, the kit containing a component for solidifying the medium.
Item 13. A method for producing a pneumococcal sample for use in a pneumococcal antibody sample evaluation test, the method comprising a step of culturing a pneumococcus in the medium according to any one of Items 1 to 10 to obtain a pneumococcal sample.
Item 13A. Use of a solid medium in production of a medium for preparing a pneumococcal sample for use in a pneumococcal antibody sample evaluation test.
Item 13B. Use of a solid medium as a medium for preparing a pneumococcal sample for use in a pneumococcal antibody sample evaluation test.
Item 14. The production method according to Item 13, wherein the evaluation test evaluates at least one property selected from the group consisting of a binding property of the pneumococcal antibody sample to the pneumococcal sample, a complement deposition ability based on the binding, and a bactericidal property based on the complement deposition.
Item 15. A pneumococcal sample for use in a pneumococcal antibody sample evaluation test, the sample being obtained by the production method according to Item 13 or 14.
Item 16. A pneumococcal antibody sample evaluation method comprising a step of contacting the pneumococcal sample according to Item 15 with a pneumococcal antibody sample.
Item 17. The evaluation method according to Item 16, further comprising a step of evaluating at least one property selected from the group consisting of a binding property of the pneumococcal antibody sample to the pneumococcal sample, a complement deposition ability based on the binding, and a bactericidal property based on the complement deposition.

### Advantageous Effects of Invention

According to the present invention, by using, as a pneumococcal sample for use in a pneumococcal antibody sample evaluation test, a pneumococcal sample obtained by culturing a pneumococcus in a solid medium, the absolute detection value and/or the ratio of the detection value to a reference in the pneumococcal antibody sample evaluation test can be further increased, and/or pneumococcal antibody samples against a wider variety of pneumococcal strains can be evaluated.

### Brief Description of Drawings

Fig. 1 shows anti-PspA IgG antibody titers in rabbit PspA3+2 immune serum against clades 1 to 5 of PspAs (Test Example 1) .
Fig. 2 shows the results obtained by measuring antibody binding and complement deposition in pneumococcal strains derived from adults with IPD using rabbit anti-PspA3+2 immune serum (Test Example 2).
Fig. 3 shows the results obtained by measuring complement deposition activity against various types of strains derived from adults with IPD using rabbit PspA3+2 immune serum (Test Example 3).
Fig. 4 shows the results obtained by measuring opsonic activity against pneumococcal strains derived from adults with IPD using rabbit anti-PspA3+2 immune serum (Test Example 4).
Fig. 5 shows an anti-capsular antibody titer against a capsular antigen of each serotype contained in a vaccine in rabbit anti-PCV13 immune serum (Test Example 5).
Fig. 6 shows the results obtained by measuring opsonic activity against pneumococcal strains derived from adults with IPD using rabbit anti-PCV13 immune serum (Test Example 6).
Fig. 7A shows the results obtained by measuring antibody binding in pneumococcal strains derived from adults with IPD using rabbit anti-PspA3+2 immune serum (Test Example 7).
Fig. 7B shows the results obtained by measuring complement deposition in pneumococcal strains derived from adults with IPD using rabbit anti-PspA3+2 immune serum (Test Example 7).
Fig. 7C shows the results obtained by measuring opsonic activity in pneumococcal strains derived from adults with IPD using rabbit anti-PspA3+2 immune serum (Test Example 7).

### Description of Embodiments

In this specification, the expressions "comprise" and "contain" encompass the concepts of "comprise," "contain," "consist essentially of," and "consist of."

### 1. Medium for Preparing Pneumococcal Sample

In one aspect, the present invention relates to a medium for preparing a pneumococcal sample for use in a pneumococcal antibody sample evaluation test (in this specification, sometimes referred to as the "medium of the present invention"), wherein the medium contains a solid medium, which is explained below.

The solid medium is not particularly limited, as long as it is a medium in a solid state and pneumococci can be cultured.

The solid medium contains a solidification component for keeping the medium solid, as necessary. Examples of the solidification component include agar, gelatin, collagen, gellan gum, and the like. Of these, agar is preferred. The solidification components may be used singly, or in a combination of two or more.

The content of the solidification component in the solid medium is not particularly limited, and may vary depending on the type of the solidification component. The content is, for example, 0.1 to 5% by mass, preferably 0.2 to 3% by mass, more preferably 0.5 to 2.5% by mass, even more preferably 1 to 2% by mass, still more preferably 1.2 to 1.6% by mass, and still even more preferably 1.3 to 1.5% by mass.

The solid medium usually contains a nutrition component used for the growth of pneumococci. Examples of the nutrition component include a carbon source, a nitrogen source, a sulfur source, an amino acid, an inorganic salt, a growth factor, and the like. The solid medium can be a synthetic medium, a semisynthetic medium, a natural medium, or the like, depending on the type of the nutrition component. Specific examples of the nutrition component include peptones (e.g., casein peptone, meat peptone, myocardial peptone, gelatin peptone, and soya peptone); extracts (e.g., yeast extract, meat extract, fish extract, heart infusion, malt extract, potato extract, and tomato juice); sugars, such as glycerol, glucose, fructose, sucrose, and hydrolysates of molasses or starches; organic acids, such as fumaric acid, citric acid, and succinic acid; amino acids, such as alanine, arginine, asparagine, cysteine, cystine, glutamic acid, glutamine, glycine, histidine, isoleucine, leucine, lysine, methionine, phenylalanine, proline, hydroxyproline, serine, threonine, tryptophan, tyrosine, valine, or derivatives thereof; inorganic ammonium salts, such as ammonium sulfate, ammonium chloride, and ammonium phosphate; organic nitrogen sources, such as soybean hydrolysates; inorganic nitrogen sources, such as ammonia gas and aqueous ammonia; bases, such as adenine, guanine, and uracil; inorganic salts, such as sodium chloride, potassium chloride, calcium chloride, sodium phosphate, potassium phosphate, sodium carbonate, potassium carbonate, magnesium sulfate, iron ions (iron sulfate, iron nitrate, etc.), and manganese ions (sulfate manganese); various vitamins; and the like. Of these, peptones and/or extracts, inorganic salts, and vitamins are preferred. The nutrition components may be used singly, or in a combination of two or more.

The content of the nutrition component in the solid medium is not particularly limited, and may vary depending on the type of the solidification component. For example, when a peptone and/or an extract is used, the total content thereof is, for example, 5 to 40% by mass, preferably 10 to 35% by mass, more preferably 15 to 30% by mass, and even more preferably 17 to 25% by mass. When an inorganic salt is used, the total content is, for example, 1 to 10% by mass, preferably 2 to 8% by mass, more preferably 3 to 7% by mass, even more preferably 4 to 6% by mass, and still more preferably 4.5 to 5.5% by mass.

In addition to the above, the solid medium preferably contains catalase, a blood component, and the like. The blood component may include whole blood components, or a component obtained by wholly or partially removing part of blood components (e.g., a blood cell component, a coagulation factor, etc.). Examples of the blood component include whole blood components, a serum component, a plasma component, and the like; and whole blood components are preferred. Examples of animals from which the blood component is derived include various mammals, such as humans, monkeys, mice, rats, dogs, cats, rabbits, pigs, horses, cows, sheep, goats, and deer. Sheep, rabbits, horses, and humans are preferred; and sheep are more preferred. The blood components may be used singly, or in a combination of two or more.

When catalase is used, its usage is not particularly limited. For example, a solid medium obtained from a liquid medium containing catalase may be used, or a solid medium obtained by applying catalase to the surface of the solid medium may be used. In the latter case, 10 to 500 units, and preferably 50 to 200 units, of catalase may be applied per cm² of the medium surface.

When a blood component is used, its usage is not particularly limited. For example, a solid medium obtained from a liquid medium containing a blood component may be used, or a solid medium obtained by applying a blood component to the surface of the solid medium may be used. In the former case, the content of the blood component in the solid medium is not particularly limited; however, it is, for example, 0.5 to 15% by mass, preferably 2 to 10% by mass, more preferably 3 to 8% by mass, even more preferably 4 to 6% by mass, and still more preferably 4.5 to 5.5% by mass.

The solid medium usually contains a solvent such as water. The content of the solvent in the solid medium is not particularly limited; however, it is, for example, 40 to 97% by mass, preferably 70 to 95% by mass, more preferably 80 to 95% by mass, and even more preferably 90 to 95% by mass.

The solid medium may contain other components in addition to the above. The other components are not particularly limited, as long as they do not significantly inhibit the growth of pneumococci. The content of the other components in the solid medium is not particularly limited; however, it is, for example, 0 to 10% by mass, preferably 0 to 5% by mass, more preferably 0 to 2% by mass, and even more preferably 0 to 1% by mass.

The form of the solid medium is not particularly limited; and examples include a plate medium, a slope medium, a semi-slope medium, a high-layer medium, and the like. Of these, the flat medium is preferred.

The medium of the present invention can be produced according to a known method for solid media. Typically, after various components are dissolved in a solvent (usually by heat), the mixture is introduced into a container having a desired shape, and solidified, thus dead-storing the medium. In order to prevent decomposition by heat, a blood component or the like is preferably added after the components are dissolved. In the production process, if necessary, a sterilization treatment (e.g., an autoclave treatment) is performed as an integrated treatment with the heat treatment for dissolving the components, or separately from the heat treatment.

The medium of the present invention is a medium used for preparing a pneumococcal sample for use in a pneumococcal antibody sample evaluation test. More specifically, the medium of the present invention is used for obtaining a pneumococcal sample by culturing pneumococci according to the method described in section "2. Pneumococcal Sample" below.

The pneumococcal antibody sample is not particularly limited, as long as it can contain a pneumococcal antibody (an antibody having a binding property (preferably, a specific binding property) to a pneumococcus). Examples of the pneumococcal antibody sample include a sample collected from a subject to which a pneumococcal vaccine has been administered, and a sample collected from a subject that may have been infected with pneumococci.

The pneumococcal vaccine is not particularly limited, as long as it is a vaccine in which a constituent component having pneumococcal antigenicity is an antigen. Examples of the antigen include those derived from pneumococci. Preferable examples include pneumococcal surface/intracytoplasmic proteins (e.g., PspA: pneumococcal surface protein A; pneumococcal histine triad (Pht) family: PhtA, PhtB, PhtD, and PhtE; pneumococcal choline-binding protein A (CbpA); pneumococcal surface adhesion A (PsaA); Pilus family proteins; pneumolysin (Ply); autolysin (LytA) neumoraminidase; IgA1 protease; protein required for cell wall separation (PcsB); serine-threonine protein kinase (StkP)); pneumococcal capsular components (e.g., polysaccharides); and the like. Pneumococcal surface/intracytoplasmic proteins are more preferred, and PspA is even more preferred. The pneumococcal surface/intracytoplasmic protein may be an intrinsic protein as is; or a modified protein thereof, e.g., a modified protein in which a partial region is deleted, a modified protein in which a pneumococcal surface/intracytoplasmic protein is fused with another protein, or a modified protein in which pneumococcal surface/intracytoplasmic proteins are fused each other. Examples of the modified proteins include the fusion proteins described in WO2014/045621. The antigens of pneumococcal vaccines may be used singly, or in a combination of two or more. In particular, when the pneumococcal capsular component is contained as an antigen, various capsular components according to the serotype can be used as the antigens.

The pneumococcal vaccine may contain other components. Examples of other components include adjuvants, such as aluminum hydroxide and CpG, and antigens against other pathogens. The administration form of the pneumococcal vaccine is not particularly limited. Examples include parenteral administration, such as intraperitoneal administration, subcutaneous administration, intradermal administration, intramuscular administration, intravenous administration, intranasal administration, transdermal administration, and transmucosal administration; as well as oral administration.

The subject is not particularly limited; and examples include various mammals, such as humans, monkeys, mice, rats, dogs, cats, rabbits, pigs, horses, cows, sheep, goats, deer, and the like.

The sample collected from the subject is not particularly limited, as long as it is a biological sample that can contain a pneumococcal antibody. Examples include a sample derived from blood (blood sample); and more specific examples include whole blood, plasma, serum, and the like.

The pneumococcal antibody sample evaluation test is not particularly limited, as long as the test can evaluate the effect of the pneumococcal antibody contained in the pneumococcal antibody sample on pneumococci. Examples of the evaluation test include an evaluation test of a binding property of the pneumococcal antibody sample to the pneumococcal sample, an evaluation test of a complement deposition ability based on the binding, and an evaluation test of a bactericidal property based on the complement deposition. Examples of the evaluation test of a complement deposition ability include a complement deposition measurement test, and examples of the evaluation test of a bactericidal property include an opsonic activity measurement test. Of these, the evaluation test of a complement deposition ability and the evaluation test of a bactericidal property are preferable; and the evaluation test of a bactericidal property is more preferable.

The pneumococcal sample is not particularly limited, as long as it is a sample of pneumococci on which a pneumococcal antibody, which is used in a pneumococcal antibody sample evaluation test, acts. Specifically, the pneumococcal sample is a sample in a state before it is brought into contact with another sample (e.g., a pneumococcal antibody sample) in a pneumococcal antibody sample evaluation test. If the preparation of the pneumococcal sample include multiple culture steps, the medium of the present invention is used in the final culture step; i.e., the medium is used in the preparation of a pneumococcal sample that is used in the pneumococcal antibody sample evaluation test without being subjected to another substantial culture step. The "another substantial culture step" herein is a culture step in an environment suitable for culturing pneumococci. For example, this step is performed in a medium, at a certain temperature or higher that is suitable for culture (for example, 15°C or higher, preferably 25°C or higher, more preferably 30°C or higher, even more preferably 35°C or higher), and/or at a certain time or more that is suitable for culture (for example, 30 minutes or more, preferably 1 hour or more, more preferably 2 hours or more). Thus, the "another substantial culture step" does not include a step in which pneumococci proliferate even in a small amount, such as a suspension step in a solution, a centrifugation step, and a preparation step of a cryopreserved sample.

The pneumococci constituting the pneumococcal sample is not particularly limited. Examples include various strains, such as ASP332(3/1), ASP365(3/1), ASP162(10A/1), ASP441(3/1), ASP204(3/1), ASP406(10A/1), ASP164(3/1), ASP110(20/1), ASP286(3/1), ASP458 (7C/1), ASP264 (22F/1), ASP242 (6C/2), ASP165(3/1), ASP109(14/1), ASP205(10A/1), ASP402-1(22F/1), ASP265(33F/1), ASP336(14/1), ASP116(6B/1), ASP113(34/1), ASP234(34/1), ASP161(22F/1), ASP460(23A/1), ASP340(6A/2), ASP407(6C/2), ASP114(24F/2), ASP337(24F/2), ASP266(6A/2), ASP334(19A/3), ASP263(19A/3), ASP206(11A/4), ASP331(35B/4), ASP338(35B/4), ASP442(11A/4), ASP123(19A/3), ASP362(23F/5), ASP108(19A/3), ASP115(14/4), ASP262(19A/3), ASP111(19A/3), ASP444(19A/3), ASP335(35B/4), ASP112(15A/4), ASP443(23F/5), ASP84(19A/3), ASP363(35B/4), ASP285(11A/4), ASP64(7F/3), ASP339(7F/3), ASP364(9V/3), ASP366(15A/4), ASP240(18C/4), ASP457(15B/3), ASP107(6A/4), ASP403(6A/5), ASP163-2(37/6), ASP114, BG9739, DBL6A, L81905, BG8743, AC94, BG6692, BG8838, DBL1, Rx1, E134, EF10197, EF6796, BG9163, DBL5, WU2, EF3296, BG8090, AC122, EF5668, BG7561, BG7817, BG11703, and ATCC6303. The pneumococci constituting pneumococcal samples may be used singly, or in a combination of two or more.

In one aspect, the present invention also relates to a composition for preparing the medium of the present invention, wherein the composition contains a component for solidifying the medium. The preparation composition may be, for example, a dry composition in which components other than a solvent are mixed, a composition in which components are not completely dissolved, even though a solvent is included, or an unsolidified composition.

In another aspect, the present invention relates to a kit for preparing the medium of the present invention, wherein the kit includes a component for solidifying the medium. The preparation kit may include, for example, a dry composition in which components other than a solvent are contained in one container; or a dry composition in which components other than a solvent are contained in two or more separate containers. The preparation kit may include reagents and instruments that can be used in the preparation of the medium of the present invention.

### 2. Pneumococcal Sample

In one aspect, the present invention relates to a method for producing a pneumococcal sample for use in a pneumococcal antibody sample evaluation test, wherein the method includes the step of culturing a pneumococcus in the medium of the present invention to obtain a pneumococcal sample (in this specification, sometimes referred to as "the production method of the present invention"). In another aspect, the present invention relates to a pneumococcal sample for use in a pneumococcal antibody sample evaluation test, wherein the sample is obtained by the production method of the present invention (in this specification, sometimes referred to as "the pneumococcal sample of the present invention"). These aspects are explained below.

The pneumococci to be cultured in the production method of the present invention are not particularly limited. The various strains listed in section "1. Medium for Preparing Pneumococcal Sample" can be used. The pneumococci to be cultured may be pneumococci stored in a frozen state, or pneumococci cultured in another culture step. The pneumococci may be used singly, or in a combination of two or more.

The culture method is not particularly limited, as long as it is a culture method in a solid medium. An appropriate method may be employed according to the form of the solid medium. For example, when the solid medium is a plate medium, a method in which pneumococci are plated on the surface of the medium and cultured under conditions suitable for culture is used.

The culture temperature is not particularly limited, as long as the growth of pneumococci is not significantly inhibited. The culture temperature is, for example, 20 to 45°C, preferably 30 to 40°C, more preferably 35 to 39°C, and even more preferably 36 to 38°C.

The culture atmosphere is not particularly limited, as long as the growth of pneumococci is not significantly inhibited. Examples of the culture atmosphere include air, an atmosphere in which carbon dioxide is introduced into the atmosphere to increase the concentration thereof (e.g., 3 to 7%, preferably 4 to 6%, and more preferably 4.5 to 5.5%), and the like.

The culture time is not particularly limited, as long as the growth of pneumococci at a certain level is possible. The culture time is, for example, 30 minutes to 48 hours, preferably 1 to 24 hours, more preferably 2 to 12 hours, and even more preferably 3 to 8 hours.

A pneumococcus obtained by culture is optionally washed with PBS or the like, usually after collection. The pneumococcus as is or through another step (e.g., refrigeration) is used as the pneumococcal sample of the present invention for contact with another sample (e.g., pneumococcal antibody sample) in a pneumococcal antibody sample evaluation test without undergoing the "another substantial culture step" explained in section "1. Medium for Preparing Pneumococcal Sample."

The pneumococcal antibody sample evaluation test, pneumococcal sample, and the like are the same as those defined in section "1. Medium for Preparing Pneumococcal Sample."

### 3. Pneumococcal Antibody Sample Evaluation Method

In one aspect, the present invention relates to a pneumococcal antibody sample evaluation method (in this specification, sometimes referred to as the "evaluation method of the present invention") comprising the step of contacting the pneumococcal sample of the present invention and a pneumococcal antibody sample. An explanation is provided below.

The evaluation method of the present invention is not particularly limited, as long as the method can evaluate the effect of the pneumococcal antibody contained in the pneumococcal antibody sample on a pneumococcus. Examples of the evaluation method include the step of evaluating a binding property of the pneumococcal antibody sample to the pneumococcal sample, the step of evaluating a complement deposition ability based on the binding property, the step of evaluating a bactericidal property based on the complement deposition, and the like. Examples of the evaluation step of complement deposition ability include a complement deposition measurement step, and examples of the evaluation step of bactericidal property include an opsonic activity measurement step. Of these, the evaluation step of complement deposition ability and the evaluation step of bactericidal property are preferred, and the evaluation step of bactericidal property is more preferred.

When the pneumococcal sample to be subjected to the evaluation method of the present invention is in a frozen state, it is thawed as necessary, and then used.

The pneumococcal antibody sample to be subjected to the evaluation method of the present invention is the same as that defined in section "1. Medium for Preparing Pneumococcal Sample."

In the evaluation method of the present invention, the pneumococcal sample of the present invention is brought into contact with the pneumococcal antibody sample. In other words, the pneumococcal sample of the present invention and the pneumococcal antibody sample are placed under conditions in which they can come into contact with each other. The specific mode of the contact is not particularly limited. For example, the contact is performed by mixing the pneumococcal sample of the present invention with the pneumococcal antibody sample. At the time of contact, a blocking agent such as BSA or skim milk may be added, as necessary.

The contact time of the pneumococcal sample of the present invention and the pneumococcal antibody sample is not particularly limited, as long as they can bind to form a complex. The contact time is, for example, 5 minutes to 4 hours, preferably 15 minutes to 2 hours, more preferably 20 minutes to 1 hour. The temperature during contact is not particularly limited; however, it is, for example, 20 to 45°C, preferably 30 to 40°C, more preferably 35 to 39°C, and even more preferably 36 to 38°C.

To evaluate the binding property of the pneumococcal antibody sample to the pneumococcal sample, the amount of the complex obtained in the above contact step is measured using an appropriate label or the like. More specifically, the amount of the complex can be measured based on an antigen-antibody reaction using a labeled secondary antibody.

To evaluate complement deposition ability, a complement component is also brought into contact in the above contact step, or a complement component is further brought into contact with the complex obtained in the above contact step; and the amount of the obtained complex is measured using an appropriate label or the like. The contact conditions are the same as those in the above contact step. More specifically, the amount of the complex can be measured based on an antigen-antibody reaction using a labeled secondary antibody.

To evaluate the bactericidal property, a complement component and a phagocyte are also brought into contact in the above contact step, or a complement component and a phagocyte are further brought into contact with the complex obtained in the above contact step, followed by incubation for a certain period of time, after which the amount of viable bacterial count is measured according to a known method. The contact time is not particularly limited, as long as it is a time in which the phagocyte can exhibit bactericidal capacity. The contact time is, for example, 30 minutes to 5 hours, preferably 45 minutes to 2 hours, and more preferably 1 to 1.5 hours. Other contact conditions are the same as those in the above contact step. More specifically, the amount of viable cells can be measured, for example, by plating the liquid obtained by the above reaction on a solid medium, and counting the number of obtained colonies.

The complement component is not particularly limited. For example, a commercially available complement reagent can be used. In addition, according to the protocol for a multiplexed opsonophagocytic killing assay (UAB-MOPA, Reference 2) for antibodies against Streptococcus pneumonia, Version E.02, December 2014 (www.vaccine.uab.edu), a complement component shown on page 16 thereof can be used.

The phagocyte is not particularly limited, and may be a phagocyte that is applicable to the measurement of complement-dependent bactericidal capacity. Examples of commonly used phagocytes include, but are not limited to, human leukemia-derived HL-60 cells having induced phagocytic activity. The phagocyte is preferably a neutrophil.

According to the evaluation method of the present invention, by using a pneumococcal sample obtained by culturing a pneumococcus in a solid medium as a pneumococcal sample for use in a pneumococcal antibody sample evaluation test, the absolute detection value and/or the ratio of the detection value to a reference in the pneumococcal antibody sample evaluation test can be further increased, and pneumococcal antibody samples against a wider variety of pneumococcal strains can be evaluated.

### Examples

Hereinafter, the present invention will be described in detail based on the Examples; however, the present invention is not limited to these examples.

### Test Example 1. Measurement of Antibody Titer of Anti-PspA3+2 Rabbit Immune Serum

Anti-PspA IgG antibody titers of rabbit PspA3+2 immune serum and non-immune serum were measured by ELISA, as detailed below.

### Test Example 1-1. Preparation of Serum

According to the method of a known document (WO 2014/045621), a PspA3+2 antigen (a fusion protein of pneumococcal strain TIGR4-derived PspA (family 2, clade 3 PspA) and pneumococcal strain WU2-derived PspA (family 1, clade 2 PspA), in which a vector-derived sequence containing a polyhistidine tag, a sequence of residues 32 to 524 of the amino acid sequence of the TIGR4 PspA (Accession No. AAK74303, 744aa), a sequence corresponding to an EcoRI recognition nucleotide sequence, and a sequence of residues 32 to 409 of a partial amino acid sequence of the WU2 PspA (Accession No. AAF27710, 415aa) are connected in this order from the N-terminal) was prepared. 5.0 µg of the PspA3+2 antigen and 250 µg of aluminum hydroxide gel were mixed, and the resulting mixture was subcutaneously inoculated into a New Zealand white rabbit (weight: about 2.5 kg). Inoculation was performed a total of two times at a two-week interval. Two weeks after the final immunization, cardiac blood was collected to obtain immune serum. On the other hand, partial blood sampling from the rabbit ear vein was performed before immunization to collect non-immune serum.

### Test Example 1-2. Preparation of PspA Antigens for ELISA

Proteins individually comprising an α-helical region and a proline-rich region of each of the clade 1 to 5 PspAs were prepared as recombinant proteins, and used as PspA antigens for ELISA. A PspA from BG9739, a PspA from D39, a PspA from TIGR4, a PspA from EF5668, and a PspA from ATCC6303 were respectively used as clade 1 PspA, clade 2 PspA, clade 3 PspA, clade 4 PspA, and clade 5 PspA. Specifically, they were prepared according to the method described in the known document (WO2014/045621).

### Test Example 1-3. ELISA

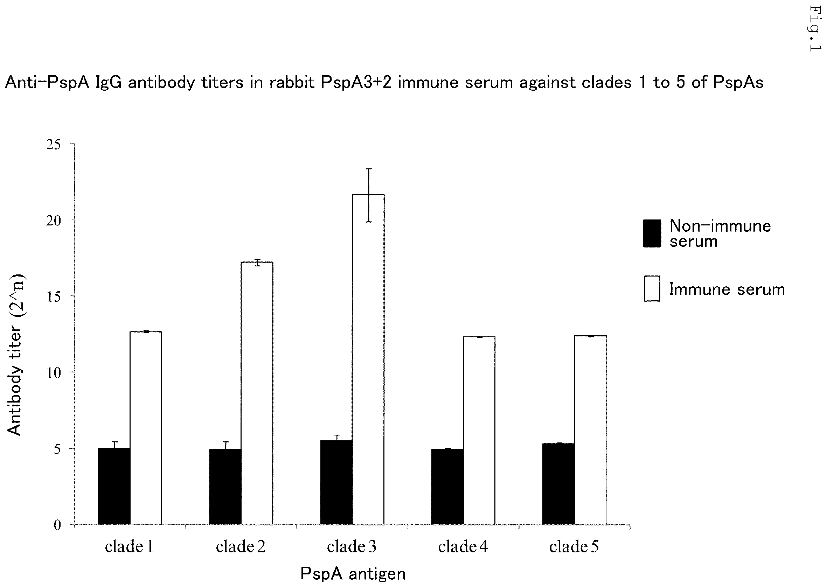

The amount of antigen-specific IgG against a PspA antigen was measured by an ELISA method. A 1 µg/ml PspA antigen for ELISA (Test Example 1-2) was added to a 96-well microplate at 100 µl/well, followed by incubation at 4°C overnight to perform solidification. The wells were washed with PBST (phosphate buffered saline containing 0.05% Tween 20). 1% BSA-PBST was added at 100 µl/well, and the microplate was allowed to stand at 37°C for 1 hour to perform blocking. The wells were washed with PBST using a microplate washer. 50 µl of a diluted solution obtained by subjecting the serum (Test Example 1-1) to serial two-fold dilution with 1% BSA-PBST was added to each well, followed by incubation at 37°C for 30 minutes. After the wells were washed with PBST, 100 µl of horseradish peroxidase (HRP) conjugated affinity-purified goat anti-rabbit IgG diluted 5000-fold was added to each well, followed by incubation at 37°C for 30 minutes. After the wells were washed with PBST, 100 µl of a chromogenic substrate was added to each well, followed by incubation at room temperature for 45 minutes under light-shielding. 100 µl of a 1.5% oxalic acid solution was added to each well, and the light was shielded until measurement. Afterward, absorbance at OD 415 nm for each well was measured. The highest dilution factor (2ⁿ) in which a value obtained by subtracting a blank from the absorbance was 0.1 or more was defined as the antibody titer. Fig. 1 shows the results.

### Test Example 1-4. Results

As shown in Fig. 1, immune serum showed an antibody titer higher than that of non-immune serum against the PspA antigen of each clade.

### Test Example 2. Evaluation of Anti-PspA 3+2 Rabbit Immune Serum 1

The serum (Test Example 1-1) was evaluated using pneumococcal samples of three clinical isolates: ASP161 (serotype: 22F/clade 1), ASP114 (serotype: 24F/clade2), and ASP337 (serotype: 24F/clade 2) in the following manner.

### Test Example 2-1. Preparation of Test Pneumococcal Sample by Liquid Medium Culture Method

Pneumococci stored at -80°C were plated on a blood agar medium (Nippon Becton Dickinson, sheep blood agar medium, 252201 252202), followed by stationary culture at 37°C, 5% CO₂, overnight. A pneumococcal single colony growing in the blood agar medium was cultured in a THY liquid medium (Todd-Hewitt broth supplemented with 0.5% yeast extract), and bacteria in the exponential growth phase were subjected to centrifugation (7500 g, 4°C, 10 minutes) to obtain a precipitate. The precipitate was washed once with a THY medium, and then suspended in a THY medium containing 20% glycerol. After measurement of the bacterial concentration, the suspension was flash-frozen in liquid nitrogen, and stored at - 80°C.

### Test Example 2-2. Preparation of Test Pneumococcal Sample by Solid Medium Culture Method

The bacteria obtained in Test Example 2-1, which were stored at -80°C, were quickly thawed at 37°C, and plated on a blood agar medium (Nippon Becton Dickinson, sheep blood agar medium, 252201 252202), followed by culture at 37°C, 5% CO₂, for 4 to 5 hours. Pneumococci growing in the blood agar medium were scraped with a platinum loop, and introduced into an Eppendorf tube on ice containing 1 ml of PBS, followed by suspension. The suspension was centrifuged (13500 g, 4°C, 2 minutes) to remove the supernatant. 1 ml of cold PBS containing 20% glycerol was added, and the pellet was sufficiently suspended. After measurement of the bacterial concentration, the suspension was flash-frozen in liquid frozen, and stored at -80°C.

### Test Example 2-3. Antibody Binding Test

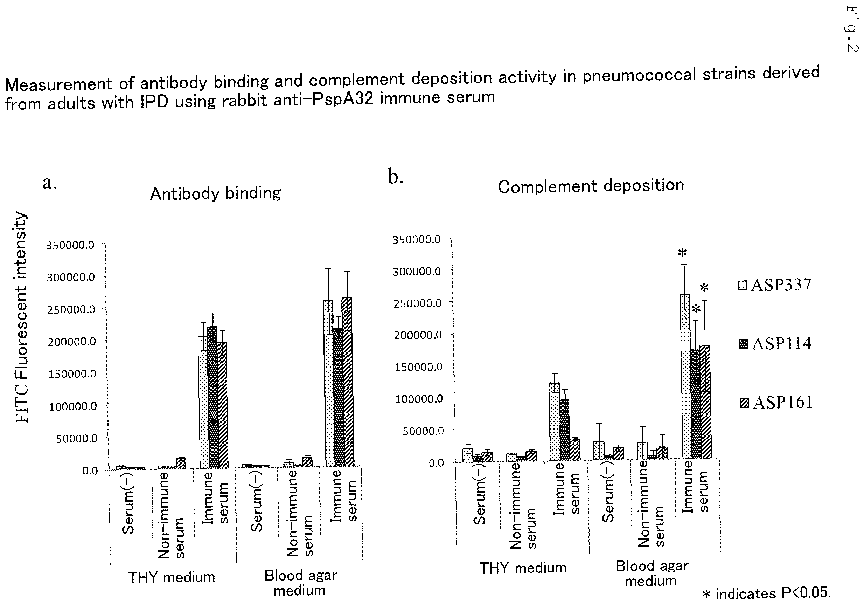

The bacteria stored at -80°C (Test Example 2-1 and Test Example 2-2) were quickly thawed in a thermostat at 37°C. The obtained pneumococcal sample (1 × 10⁶ cfu) and serum (2 µl, Test Example 1-1) were mixed. The mixture was diluted with 1% BSA-containing PBS to a total volume of 100 µl, followed by incubation at 37°C for 30 minutes. After centrifugation (13500 g, 4°C, 2 minutes), the supernatant was removed. 1 ml of cold PBS was added; afterward, centrifugation (13500 g, 4°C, 2 minutes) was performed, and the supernatant was removed. 100 µl of FITC-goat anti-rabbit IgG diluted 100-fold with PBS was added, followed by sufficient suspension. Incubation was performed for 30 minutes at 4°C under light-shielding. After centrifugation (13500 g, 4°C, 2 minutes), the supernatant was removed. 1 ml of cold PBS was added; afterward, centrifugation (13500 g, 4°C, 2 minutes) was performed, and the supernatant was removed. The pellet was suspended in 100 µl of PBS, and 100 µl of 2% formamide was further added thereto to perform sufficient suspension. The resulting suspension was analyzed by flow cytometry, and the mean fluorescent intensity (MFI) of pneumococci at 10,000 events was measured. The significant difference was evaluated using GraphPad Prism statistical software, based on the p value calculated by an unpaired t-test. The results are shown in Fig. 2a.

### Test Example 2-4. Complement Deposition Test

The bacteria stored at -80°C (Test Example 2-1 and Test Example 2-2) were quickly thawed in a thermostat at 37°C. The obtained pneumococcal sample (1 × 10⁶ cfu) and serum (2 µl, Test Example 1-1) were mixed. The mixture was diluted with 1% BSA-containing PBS to a total volume of 100 µl, followed by incubation at 37°C for 30 minutes. After centrifugation (13500 g, 4°C, 2 minutes), the supernatant was removed. 1 ml of cold PBS was added; afterward, centrifugation (13500 g, 4°C, 2 minutes) was performed, and the supernatant was removed. 100 µl of baby rabbit serum (complement source) diluted 10-fold with a complement diluent (CH50 Seiken) was added and mixed, followed by incubation at 37°C for 30 minutes. After centrifugation (13500 g, 4°C, 2 minutes), the supernatant was removed. 1 ml of cold PBS was added; afterward, centrifugation (13500 g, 4°C, 2 minutes) was performed, and the supernatant was removed. 100 µl of FITC-goat anti-rabbit IgG diluted 100-fold with PBS was added, followed by sufficient suspension. Incubation was performed for 30 minutes at 4°C under light-shielding. After centrifugation (13500 g, 4°C, 2 minutes), the supernatant was removed. 1 ml of cold PBS was added; afterward centrifugation (13500 g, 4°C, 2 minutes) was performed, and the supernatant was removed. The pellet was suspended in 100 µl of PBS, and 100 µl of 2% formamide was further added thereto to perform sufficient suspension. The resulting suspension was analyzed by flow cytometry, and the mean fluorescent intensity (MFI) of pneumococci at 10,000 events was measured. The significant difference was evaluated using GraphPad Prism statistical software, based on the p value calculated by an unpaired t-test. The results are shown in Fig. 2b.

### Test Example 2-5. Results

In the antibody binding of anti-PspA3+2 IgG to pneumococcal strains, immune serum showed antibody binding higher than that of non-immune serum (Fig. 2a) under both liquid medium culture conditions and solid medium culture conditions. The difference was not significant between the solid medium culture conditions and the liquid medium culture conditions; however, ASP337 and ASP161 strains showed a higher tendency (Fig. 2a). Regarding the level of complement deposition on pneumococcal strains via anti-PspA3+2 antibodies, immune serum showed complement deposition activity higher than that of non-immune serum under both liquid medium culture conditions and solid medium culture conditions. (Fig. 2b). In addition, in all of the three measured strains, the strains cultured in the solid medium had complement deposition activity significantly higher than that of those cultured in the liquid medium (p<0.05) (Fig. 2b).

### Test Example 3. Evaluation of Anti-PspA3+2 Rabbit Immune Serum 2

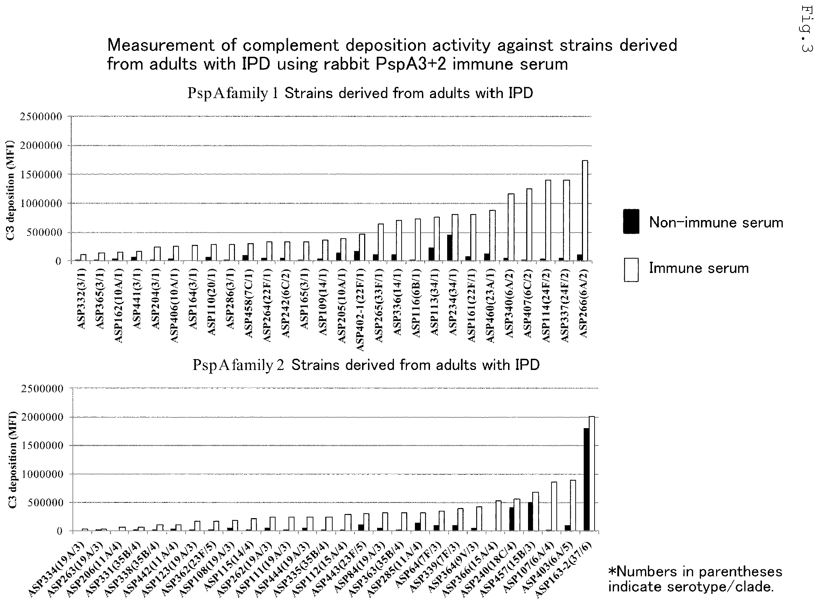

Using pneumococcal samples (samples obtained by the solid culture method) obtained by culturing various types of clinically isolated pneumococcal strains in the same manner as in Test Example 2-2, serum complement deposition activity was measured as in Test Example 2-4. The results are shown in Fig. 3.

The pneumococcal strains (56 strains) used for the measurement are pneumococcal strains derived from adults with IPD. These strains include vaccine-type serotypes and non-vaccine-type serotypes, and express various clade 1 to 5 PspAs. In all of these 56 strains, anti-PspA3+2 rabbit immune serum showed complement deposition higher than that of non-immune rabbit serum. The results indicated that the use of pneumococcal samples obtained by the solid medium culture method allowed the evaluation of immune serum against a very wide variety of pneumococcal strains.

### Test Example 4. Evaluation of Anti-PspA3+2 Rabbit Immune Serum 3

Regarding the three strains used in the antibody binding and the complement deposition, the opsonic activity via the anti-PspA antibody was measured using strains for tests in liquid medium culture and solid medium culture, as detailed below.

### Test Example 4-1. Preparation of Phagocytes

Human leukemia-derived HL-60 cells were cultured in a CM1 medium (500 ml of RPMI1640, 50 ml of inactivated FBS, 5 ml of GlutaMax-1, 5 ml of penicillin/streptomycin) at 37°C in the presence of 5% CO₂; afterward, the medium was exchanged for a medium for differentiation (500 ml of RPMI 1640, 50 ml of inactivated FBS, 5 ml of GlutaMax-1, and 4.55 ml of DMF (dimethyl formamide)) to perform differentiation induction. 4 Days to 5 days after the start of the differentiation culture, the HL-60 cells were washed with HBSS (without Ca⁺⁺, Mg⁺⁺), and then washed with HBSS (with Ca⁺⁺, Mg⁺⁺). The obtained cells were suspended at 1 × 10⁷ cells/ml in an OBB solution (32 ml of distilled water, 4 ml of 10 × HBSS (with Ca⁺⁺, Mg⁺⁺), 4 ml of 1% gelatin, 2.12 ml of inactivated FBS) before use.

### Test Example 4-2. Opsonic Activity Measurement Test

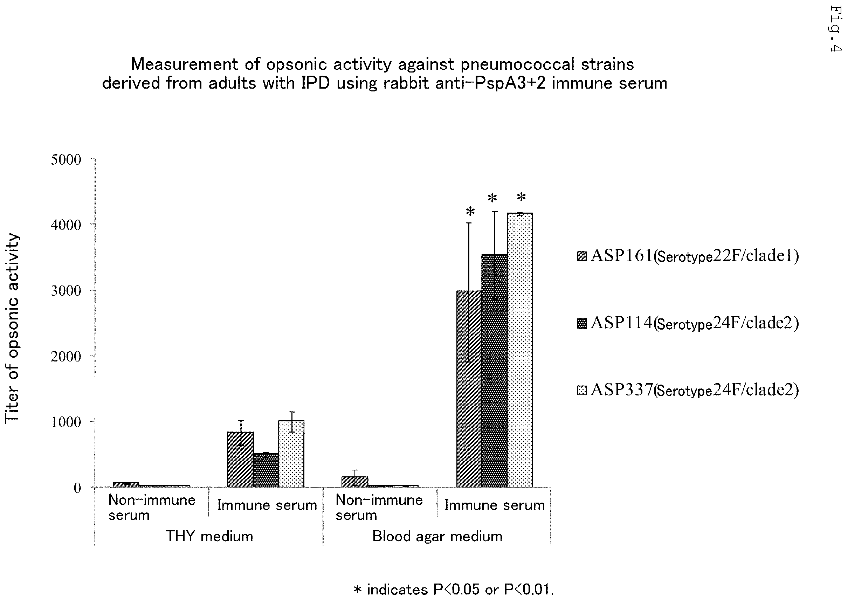

Pneumococcal samples obtained by culturing pneumococcal strains derived from adults with IPD in the same manner as in Test Example 2-1 or 2-2 were used. Pneumococcal sample 500CFU was seeded in each well of a 96-well microplate. 20 µl of the serum (Test Example 1-1) or a serial dilution thereof was added to each well, followed by incubation at room temperature for 30 minutes. 10 µl of a complement component (Pel-Freez 3-4 Week Baby Rabbit Complement, Sterile (Pel-Freez)) (final concentration 0.75%) and phagocytes cells (Test Example 4-1) were added in an amount of 4 × 10⁵ cells, followed by incubation at 37°C for 75 minutes in the presence of 5% CO₂. The reaction solution in the well was added to an agar medium, followed by culture at 37°C for 16 hours in the presence of 5% CO₂. The number of pneumococcal colonies appearing in the agar medium was counted to calculate the concentration of the serum at which 50% of the pneumococci was killed, thus measuring the opsonic activity. The significant difference was evaluated using GraphPad Prism statistical software, based on the p value calculated by an unpaired t-test. The results are shown in Fig. 4.

### Test Example 4-3. Results

Regarding the level of opsonic activity against the pneumococcal strain via the anti-PspA3+2 antibody, immune serum showed opsonic activity higher than that of non-immune serum under both liquid medium culture conditions and solid medium culture conditions (Fig. 4). Further, significantly high opsonic activity was achieved under solid medium culture conditions compared to liquid medium culture conditions; and the opsonic activity against the ASP161 strain, the ASP114 strain, and the ASP337 strain under the solid medium culture conditions is respectively 3.6 times (p <0.05), 7 times (p <0.01), and 4 times (p <0.01) higher than that under liquid medium conditions (Fig. 4) .

### Test Example 5. Measurement of Antibody Titer of Anti-PCV13 Rabbit Immune Serum

Antibody titers of anti-PCV13 rabbit immune serum against 13 types of capsular antigens contained in PCV13 were measured by an ELISA method, as detailed below.

### Test Example 5-1. Preparation of Serum

A roughly 2.5 kg New Zealand white rabbit was immunized subcutaneously with current commercially available vaccine PCV13 at 1/10 of the human dose a total of two times at a two-week interval. Two weeks after the final immunization, cardiac blood was collected to obtain immune serum. On the other hand, partial blood sampling from the rabbit ear vein was performed before immunization to collect non-immune serum.

### Test Example 5-2. ELISA

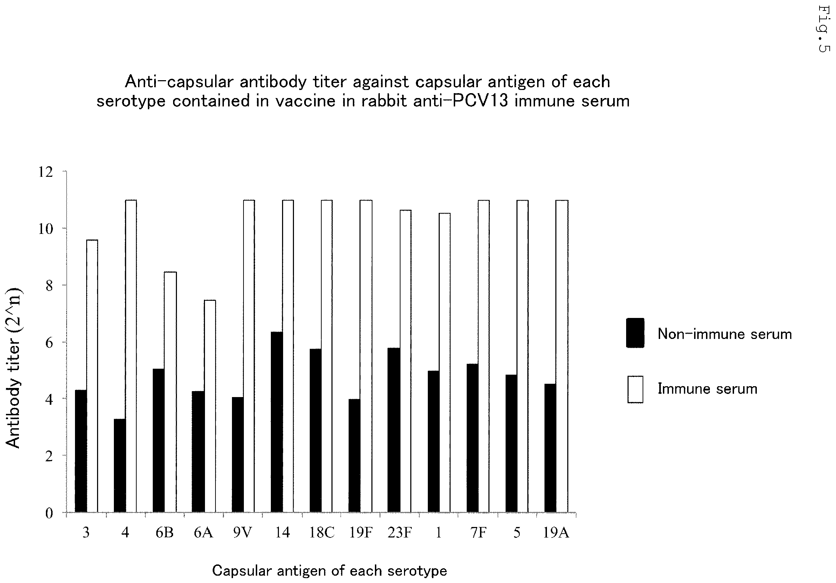

Measurement was conducted in the same manner as in Test Example 1-3, except that a 5 µg/ml capsular antigen of each serotype (obtained from ATCC) was used as an antigen solution for immobilization. The results are shown in Fig. 5.

### Test Example 5-3. Results

The results indicated that anti-PCV13 rabbit immune serum contained antibodies having high antibody titers against 13 types of capsules (Fig. 5).

### Test Example 6. Evaluation of Anti-PCV13 Rabbit Immune Serum

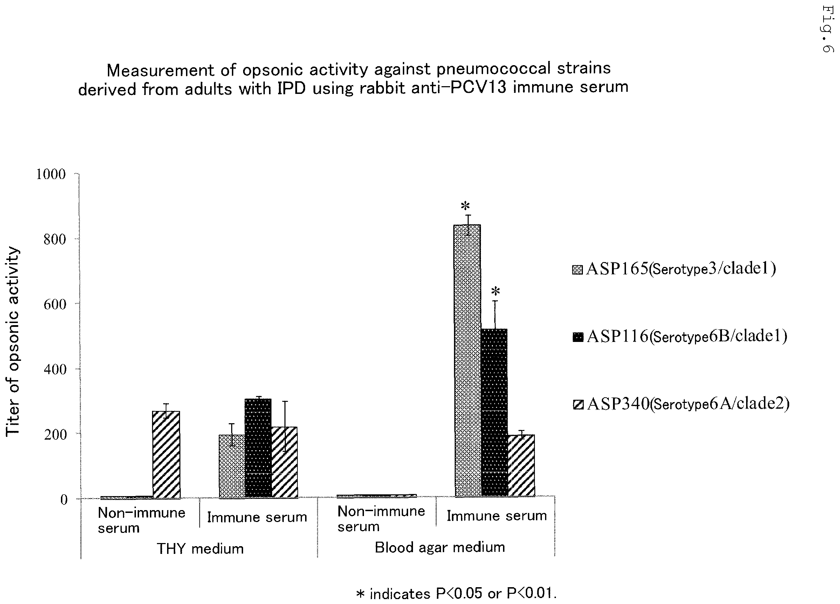

Opsonic activity was measured in the same manner as in Test Example 4-2, except that three strains, i.e., ASP165 (serotype 3/clade 1), ASP116 (serotype 6B/clade 1), and ASP340 (serotype 6A/clade 2) were used, and anti-PCV13 rabbit immune serum (Test Example 5-1) was used as immune serum. The results are shown in Fig. 6.

Regarding the level of opsonic activity against the pneumococcal strain via the anti-PCV13 antibody, the opsonic activity of immune serum against ASP165 and ASP116 strains was higher than that of non-immune serum under both liquid medium culture conditions and solid medium culture conditions. In contrast, regarding the opsonic activity against ASP340, there was no difference between the serum before and after immunization under liquid medium culture conditions; however, immune serum showed opsonic activity higher than that of non-immune serum under solid medium culture conditions. The ASP340 strain is serotype 6A. Despite the fact that the anti-capsular 6A antibody titer in anti-PCV13 immune serum is significantly higher than that before immunization, absolutely no difference in opsonic activity may be attributable to the efficiency of the evaluation method. In addition, regarding the level of opsonic activity against the pneumococcal strain via the anti-PCV13 antibody, significantly high opsonic activity against ASP165 (p<0.01) and ASP116 (p<0.05) strains was attained in solid culture, as compared to that in liquid culture. The results indicate that the solid medium culture method is a more efficient and appropriate method than the liquid medium culture method, even in the evaluation of the efficacy of capsular antibodies.

### Test Example 7. Evaluation of Anti-PspA3+2 Rabbit Immune Serum 4

Evaluation tests were performed using test pneumococcal samples prepared in solid media having different components. Specifically, the evaluation tests were performed as follows.

### Test Example 7-1. Preparation of Test Pneumococcal Sample

The bacteria obtained in Test Example 2-1, which were stored at -80°C, were quickly thawed at 37°C. Then, the bacteria were plated on a medium obtained by applying catalase (6,300 U/20 ml) to the surface of a THYA agar medium (Todd-Hewitt yeast extract agar, bottom area: about 55 cm²) or a blood agar medium containing 5% sheep blood, followed by culture at 37°C, 5% CO₂, for 4 to 5 hours. The other procedures were performed according to Test Example 2-2.

### Test Example 7-2. Antibody Binding Test

The serum (Test Example 1-1) in an amount of 2 µl, 1 µl, 0.5 µl, 0.25 µl, 0.125 µl, 0.0625 µl, 0.03125 µl, or 0 µl was mixed with the pneumococcal sample (Test Example 7-1) (1 × 10⁶ cfu). The other procedures were performed according to Test Example 2-3.

### Test Example 7-3. Complement Deposition Test

The serum (Test Example 1-1) in an amount of 1 µl, 0.5 µl, 0.25 µl, or 0 µl was mixed with the pneumococcal sample (Test Example 7-1) (1 × 10⁶ cfu). The other procedures were performed according to Test Example 2-4.

### Test Example 7-4. Opsonic Activity Measurement Test

The test was performed in the same manner as in Test Example 4-2, except that the pneumococcal sample obtained in Test Example 7-1 was used as a pneumococcal sample.

### Test Example 7-5. Results

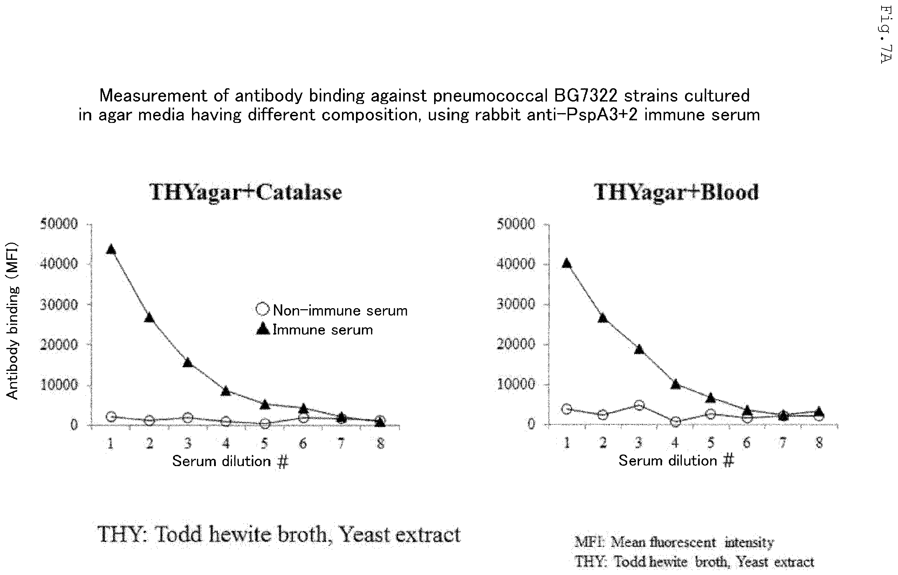

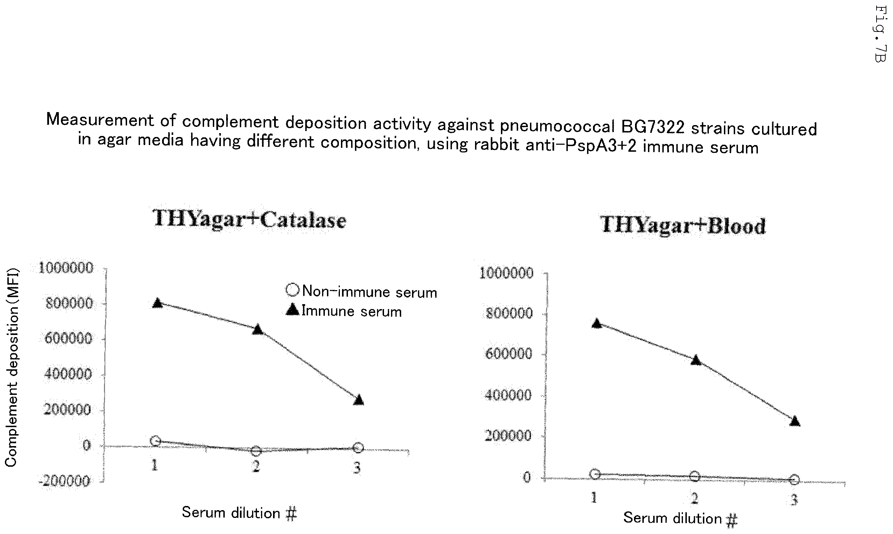

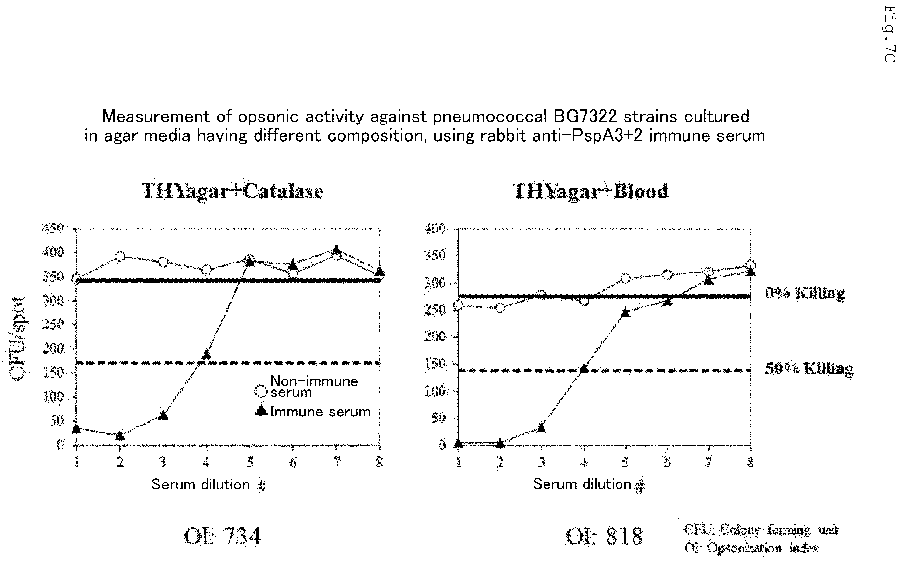

The results of Test Examples 7-2, 7-3, and 7-4 are shown in Figs. 7A, 7B, and 7C, respectively. Figs. 7A to 7C indicated that difference in components of agar media in the preparation of test pneumococcal samples did not significantly affect the antibody binding ability, complement deposition activity, and opsonic activity.

### Summary of Test Examples 1 to 6

In the present invention, significantly high activity in the complement deposition system and the opsonic activity measurement system was attained under solid medium culture conditions, as compared to liquid medium culture conditions. PspA immune serum and PCV13 immune serum also showed significantly high activity under solid medium culture conditions. Further, complement deposition activity via the PspA antibody could be measured against all of the clinically isolated strains. It is considered that an increase in measurement efficiency allows accurate evaluation of serum having low vaccine effects and serum having high vaccine effects.

As described above, it was found that the use of the pneumococcal sample prepared by the solid medium culture method can further increase the sensitivity of a pneumococcal antibody sample evaluation test, and can evaluate the pneumococcal antibody sample against a wider variety of pneumococcal strains. This ensures more simple and efficient evaluation of efficacy of pneumococcal vaccines.

## Claims

1. A medium for preparing a pneumococcal sample for use in a pneumococcal antibody sample evaluation test, the medium containing a solid medium.

2. The medium according to claim 1, wherein the solid medium contains agar.

3. The medium according to claim 1 or 2, wherein the solid medium contains a blood component and/or catalase.

4. The medium according to any one of claims 1 to 3, wherein the pneumococcal antibody sample is collected from a subject to which a pneumococcal vaccine has been administered.

5. The medium according to claim 4, wherein the sample is a blood sample.

6. The medium according to claim 4 or 5, wherein the pneumococcal vaccine contains at least one member selected from the group consisting of pneumococcal surface/intracytoplasmic proteins and pneumococcal capsular components as an antigen.

7. The medium according to any one of claims 4 to 6, wherein the pneumococcal vaccine contains a pneumococcal surface/ intracytoplasmic protein as an antigen.

8. The medium according to any one of claims 1 to 7, wherein the evaluation test evaluates at least one property selected from the group consisting of a binding property of the pneumococcal antibody sample to the pneumococcal sample, a complement deposition ability based on the binding, and a bactericidal property based on the complement deposition.

9. The medium according to claim 8, wherein the evaluation test evaluates at least one property selected from the group consisting of the complement deposition ability and the bactericidal property.

10. The medium according to claim 8 or 9, wherein the evaluation test evaluates the bactericidal property.

11. A composition for preparing the medium according to any one of claims 1 to 10, the composition containing a component for solidifying the medium.

12. A kit for preparing the medium according to any one of claims 1 to 10, the kit containing a component for solidifying the medium.

13. A method for producing a pneumococcal sample for use in a pneumococcal antibody sample evaluation test, the method comprising a step of culturing a pneumococcus in the medium according to any one of claims 1 to 10 to obtain a pneumococcal sample.

14. The production method according to claim 13, wherein the evaluation test evaluates at least one property selected from the group consisting of a binding property of the pneumococcal antibody sample to the pneumococcal sample, a complement deposition ability based on the binding, and a bactericidal property based on the complement deposition.

15. A pneumococcal sample for use in a pneumococcal antibody sample evaluation test, the sample being obtained by the production method according to claim 13 or 14.

16. A pneumococcal antibody sample evaluation method comprising a step of contacting the pneumococcal sample according to claim 15 with a pneumococcal antibody sample.

17. The evaluation method according to claim 16, further comprising a step of evaluating at least one property selected from the group consisting of a binding property of the pneumococcal antibody sample to the pneumococcal sample, a complement deposition ability based on the binding, and a bactericidal property based on the complement deposition.
